# FASCICULE DE BREVET EUROPEEN

(11) **EP 3 433 609 B1**
(45) Date de publication et mention de la délivrance du brevet: **20.10.2021**
(21) Numéro de dépôt: 17715519.9
(22) Date de dépôt: 14.03.2017
(51) Int. Cl.: G01N 33/48, B01L 3/00, G01N 1/34

(54) **DISPOSITIF ET PROCÉDE DE ANALYSER D'UN ECHANTILLON SANGUIN UNTILISANT DES EMZYMES SOUS FORME LYOPHILISÉE**
VORRICHTUNG UND VERFAHREN ZUM ANALYSIEREN EINER BLUTPROBE MIT GEFRIERTROCKNETEN ENZYMEN
DEVICE AND METHOD OF ANALYSING A BLOOD SAMPLE USING LYOPHILIZED ENZYMES

(30) Priorité: 21.03.2016 FR 1652415
(43) Date de publication de la demande: 30.01.2019
(73) Titulaire: Commissariat à l'Energie Atomique et aux Energies Alternatives, 75015 Paris (FR)
(72) Inventeur: CAILLAT, Patrice, 38180 SEYSSINS (FR); BOURDAT, Anne-Gaëlle, 38260 NANTOIN (FR); BRUN, Virginie, NOYAREY 38360 (FR); GILQUIN, Benoît, 38120 SAINT-EGREVE (FR)
(74) Mandataire: INNOV-GROUP
(86) Numéro de dépôt international: PCT/FR2017/050583
(87) Numéro de publication internationale: WO 2017/162956

(56) Documents cités:
- WO-A2-2007/112114
- US-A1- 2004 175 822

## Description

### Domaine technique de l'invention

La présente invention se rapporte à un dispositif de préparation d'un échantillon sanguin et à un système employant ledit dispositif pour la préparation d'un échantillon sanguin. L'invention se rapporte également à un procédé de préparation d'un échantillon sanguin mis en œuvre avec ledit dispositif.

### Etat de la technique

L'analyse protéomique basée sur la spectrométrie de masse pour détecter et doser des protéines dans des échantillons biologiques comme le plasma est de plus en plus répandue car particulièrement performante. Cependant, pour que cette analyse soit optimale et fiable, elle nécessite que l'échantillon sanguin analysé soit parfaitement stable. Or il s'avère par exemple que la durée qui s'écoule entre la collecte d'un échantillon sanguin, sa préparation (pour l'obtention de la fraction sérique ou plasmatique) et son transfert vers un laboratoire d'analyse est très variable, donnant ainsi des résultats d'analyse de qualités diverses. En effet, le processus naturel de dégradation des protéines d'intérêt présentes dans le plasma peut être plus ou moins avancé selon la durée qui s'écoule entre la collecte de l'échantillon et son transfert vers le laboratoire d'analyse, ce qui entraîne une certaine variabilité dans les résultats obtenus.

La demande de brevet US2004/175822A1 décrit une méthode pour automatiser la dégradation d'Edman via l'utilisation d'une carte microfluidique. Elle nécessite de déposer dans le séquenceur un peptide ou une protéine pure (ou très fortement enrichie) pour permettre la détermination de la séquence protéique en acide aminé par digestion chimique d'une des extrémités. En amont de cette phase il est donc nécessaire de séparer les protéines (sur un gel par exemple) et de les introduire une par une dans l'appareil.

Le but de l'invention est de proposer un dispositif qui est employé pour préparer et stabiliser un échantillon sanguin, ce dispositif permettant de traiter efficacement un échantillon sanguin en réduisant la durée qui s'écoule entre le prélèvement sanguin et la stabilisation de l'échantillon, de manière à obtenir des résultats de bonne qualité et parfaitement reproductibles. La solution de l'invention permet en outre de conserver tout au long du processus de préparation la complexité du protéome plasmatique (plusieurs centaines d'espèces distinctes de protéines).

### Exposé de l'invention

Ce but est atteint par un dispositif de préparation d'un échantillon sanguin, comprenant une carte micro-fluidique qui comporte :
- Un canal d'injection par lequel peut être injecté ledit échantillon sanguin,
- Une première chambre dans laquelle débouche ledit canal d'injection, ladite première chambre étant dédiée à la mise en oeuvre d'une opération de séparation et/ou d'extraction de protéines à analyser présentes dans ledit échantillon sanguin,
- Une deuxième chambre reliée à ladite première chambre de manière à recevoir un premier échantillon comportant des protéines d'espèces distinctes, ladite deuxième chambre étant dédiée à la mise en oeuvre d'une opération de digestion des protéines à analyser présentes en vue d'obtenir un deuxième échantillon incluant des peptides digérés et des protéines non digérées,
- Une troisième chambre reliée à ladite deuxième chambre de manière à recevoir ledit deuxième échantillon incluant les peptides digérés et les protéines non digérées, ladite troisième chambre étant dédiée à la mise en œuvre d'une opération de purification et de stabilisation des peptides digérés.

Contrairement à l'état de la technique cité ci-dessus, le but de l'invention est différent car il n'y a pas de séparation individuelle. Le dispositif de l'invention permet de préparer un échantillon sanguin en conservant la complexité du protéome plasmatique (plusieurs centaines de protéines d'espèces distinctes), et de les préparer à leur analyse ultérieure en restant global et en une seule passe. Cette complexité conservée de l'échantillon protéique est incompatible avec la méthode d'Edman car à chaque cycle du séquençage, plusieurs acides aminés seraient identifiés simultanément ce qui empêcherait toute identification.

Selon une particularité, le dispositif comporte un support de déplétion présent dans la première chambre pour capturer des protéines majoritaires présentes dans ledit échantillon sanguin.

Selon une configuration, le support de déplétion comporte des billes présentes dans la première chambre et greffées avec un ou plusieurs composants de capture adaptés aux protéines majoritaires à capturer.

Selon une autre configuration, le support de déplétion comporte des piliers positionnés dans la première chambre et greffés avec un ou plusieurs composants de capture adaptés aux protéines majoritaires à capturer.

Selon une autre particularité, la première chambre est par exemple réalisée sous la forme d'un serpentin dont les surfaces fonctionnalisées forment le support de déplétion.

Selon une variante de réalisation, le dispositif comporte un support d'enrichissement présent dans la première chambre pour fixer et enrichir les protéines à analyser présentes dans l'échantillon sanguin. Le support d'enrichissement comporte par exemple des billes présentes dans la première chambre et greffées avec un ou plusieurs composants d'enrichissement adaptés aux protéines à enrichir.

Selon une variante de réalisation, le support d'enrichissement comporte des piliers positionnés dans la première chambre et greffées avec un ou plusieurs composants d'enrichissement adaptés aux protéines à enrichir.

Selon une autre variante possible de réalisation, la première chambre est réalisée sous la forme d'un serpentin dont les surfaces forment le support d'enrichissement.

Selon une particularité, la deuxième chambre comporte une surface recouverte au moins partiellement d'une enzyme ou d'un mélange d'enzymes pour la mise en œuvre de l'opération de digestion.

En variante de réalisation, la deuxième chambre comporte un volume interne destiné à stocker une enzyme ou un mélange d'enzymes sous forme lyophilisée pour la mise en œuvre de l'opération de digestion.

Selon une variante de réalisation, le dispositif peut comporter une chambre intermédiaire de stabilisation située en aval de ladite première chambre et en amont de la deuxième chambre et destinée à la stabilisation des protéines extraites dans la première chambre.

Selon une autre variante de réalisation, le dispositif peut aussi comporter une chambre initiale de séparation, située en amont de la première chambre lorsque la première chambre est dédiée à l'extraction de protéines.

Selon une autre variante de réalisation, le dispositif peut, selon sa configuration, comporter des moyens de séparation spécifiques agencés pour séparer le plasma et les cellules sanguines présentes dans l'échantillon sanguin, agencés en amont de la première chambre.

Selon une particularité, le dispositif comporte une colonne de chromatographie liquide en phase inverse logée dans la troisième chambre pour accrocher, stabiliser et/ou séparer les peptides digérés.

De manière avantageuse, le dispositif comporte plusieurs canaux secondaires réalisées dans la carte et répartis pour déboucher dans chaque chambre.

L'invention concerne également un système de préparation d'un échantillon sanguin comportant un automate programmable, qui comporte un dispositif de préparation d'un échantillon sanguin tel que défini ci-dessus. L'automate programmable comporte une unité de traitement destinée à exécuter des modules logiciels adaptés pour générer des commandes à destination d'actionneurs selon une séquence déterminée en fonction des différentes étapes de préparation de l'échantillon sanguin sur ledit dispositif.

L'invention concerne également un procédé de préparation d'un échantillon sanguin, mis en œuvre dans le dispositif de préparation tel que défini ci-dessus. Ce procédé comporte les étapes suivantes :
- Extraction dans la première chambre de protéines à analyser présentes dans ledit échantillon sanguin et/ou séparation pour séparer le plasma des cellules sanguines dans l'échantillon sanguin,
- Digestion dans la deuxième chambre des protéines à analyser présentes dans un premier échantillon comportant des protéines d'espèces distinctes à analyser en vue d'obtenir un deuxième échantillon incluant des peptides digérés et des protéines non digérées,
- Séparation dans la troisième chambre entre les peptides digérés et les protéines non digérées.

Selon une particularité, l'étape d'extraction est réalisée par déplétion des protéines majoritaires du plasma ou enrichissement des protéines à analyser.

Selon une autre particularité, l'étape de stabilisation est avantageusement mise en œuvre par chromatographie liquide sur une colonne de type phase inverse.

### Brève description des figures

D'autres caractéristiques et avantages vont apparaître dans la description détaillée qui suit faite en regard des dessins annexés dans lesquels :
- La figure 1 représente un diagramme montrant les différentes étapes d'un procédé de préparation d'un échantillon sanguin.
- Les figures 2A à 2E représentent plusieurs variantes de réalisation d'un système de préparation d'un échantillon sanguin, incluant un dispositif de préparation de l'échantillon sanguin selon l'invention.
- Les figures 3A et 3B représentent deux chromatogrammes, issus d'une analyse par spectrométrie de masse réalisée sur des protéines issues respectivement d'une préparation réalisée en partie sur le dispositif de l'invention et d'une préparation réalisée manuellement et de manière classique.

### Description détaillée d'au moins un mode de réalisation

L'invention concerne notamment un dispositif de préparation d'un échantillon sanguin, avant son analyse, par exemple par chromatographie liquide associée à la spectrométrie de masse quantitative. Le dispositif de l'invention permettra notamment de préparer et de stocker un échantillon stabilisé destiné à une analyse ultérieure, sans risque de dégradation de l'échantillon.

Par échantillon stabilisé, on entend notamment un échantillon non sensible à une action de protéase.

Dans la suite de la description, de manière non limitative, le dispositif de l'invention sera décrit pour une préparation à partir d'un échantillon plasmatique issu de l'échantillon sanguin prélevé sur le patient, mais il faut comprendre qu'il pourrait être employé pour une préparation à partir du sérum issu de l'échantillon.

Dans la suite de la description, on entend par chambre un espace définissant un volume fermé non nul ou partiellement fermé dans lequel peut déboucher un ou plusieurs canaux.

Le dispositif comporte principalement une carte micro-fluidique C qui inclut des canaux et plusieurs chambres contribuant chacune à la préparation de l'échantillon sanguin avant analyse. Ce dispositif pourra être employé dans un système plus global de préparation sanguin. Ce système comporte notamment un automate programmable, référencé A, qui commande la préparation de l'échantillon sanguin sur la carte C.

Comme décrit ci-dessus, le dispositif de l'invention comporte une carte micro-fluidique C, ayant par exemple le format d'une carte de crédit, dans laquelle toutes les étapes menant à la préparation et à la stabilisation de l'échantillon avant son analyse peuvent être menées.

La carte microfluidique C est par exemple placée dans un logement adapté de l'automate programmable A. L'automate programmable A comporte avantageusement une unité de traitement agencée pour commander toutes les étapes nécessaires à la préparation de l'échantillon sanguin sur ladite carte. L'unité de traitement exécute des modules logiciels destinés chacun à commander une ou plusieurs de ces étapes. Ces modules logiciels sont exécutés pour générer des commandes de sortie à destination de différents actionneurs. Ces actionneurs peuvent être par exemple des micro-pompes, des systèmes d'agitation magnétique, des systèmes de chauffage, des systèmes de refroidissement, des systèmes d'émission d'ultrasons ou micro-ondes, des vannes micro-fluidiques. Ces différents actionneurs font par exemple partie du système de l'invention employé pour la préparation de l'échantillon sanguin dans la carte.

Cette carte micro-fluidique C est par exemple réalisée à l'aide de deux plaques collées l'une sur l'autre et sur chacune desquelles sont formés les motifs formant les chambres et les chemins fluidiques de la carte. La carte C est par exemple réalisée dans un matériau transparent de type PMMA, polycarbonate ou COC (pour "Cylcic Olefin Copolymer"). La carte microfluidique C sera décrite ci-après en liaison avec les différentes étapes de préparation d'un échantillon sanguin définies ci-dessous.

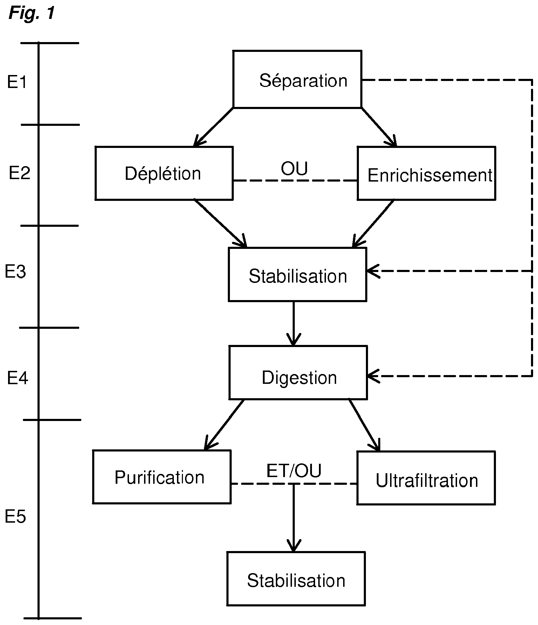

En référence à la figure 1, la carte microfluidique C est agencée pour mettre en œuvre les étapes de préparation de l'échantillon. Selon les étapes qui sont mises en œuvre, la carte microfluidique C pourra prendre différentes structures micro-fluidiques.

En référence aux figures annexées, la carte microfluidique C comporte principalement :
- Un canal d'injection IN par lequel peut être injecté ledit échantillon sanguin ;
- Plusieurs chambres successives reliées entre elles par des canaux ;
- Des canaux secondaires S1, S2 d'injection ou d'extraction reliées aux différentes chambres, par exemple pour injecter des solutions de lavage ou extraire des déchets des chambres ;

Des vannes positionnées de manière adaptée sur la carte C sont commandées par l'automate programmable A pour contrôler les différentes étapes de préparation de l'échantillon sanguin sur la carte.

Par ailleurs, des micro-pompes (non représentées) sont également commandées par l'automate programmable 2 pour faire passer l'échantillon d'une chambre à l'autre, par aspiration ou injection.

L'automate programmable 2 comporte ainsi plusieurs modules de sortie destinés chacun à la commande d'un actionneur distinct, tel que chaque vanne V ou chaque micro-pompe.

De manière plus précise, le canal d'injection IN est destiné à recevoir l'échantillon sanguin prélevé sur le patient. Le tube renfermant l'échantillon sanguin sera par exemple connecté sur l'entrée du canal d'injection IN. Cette entrée pourra être réalisée selon différentes variantes de réalisation selon le type de connexion fluidique choisie. Différents types de connexion pourront en effet être envisagés, employant par exemple un module de type « Saf-T Wing » (marque déposée). L'injection de l'échantillon sanguin dans le canal d'injection IN sera par exemple contrôlée par une première vanne commandée par l'automate et activée par une micro-pompe commandée par l'automate. L'échantillon sanguin initial prélevé au bras du patient sera par exemple de 2 à 10 millilitres. L'analyse sera effectuée sur quelques microlitres à 50 µl de plasma (ou de sérum) contenu dans cet échantillon.

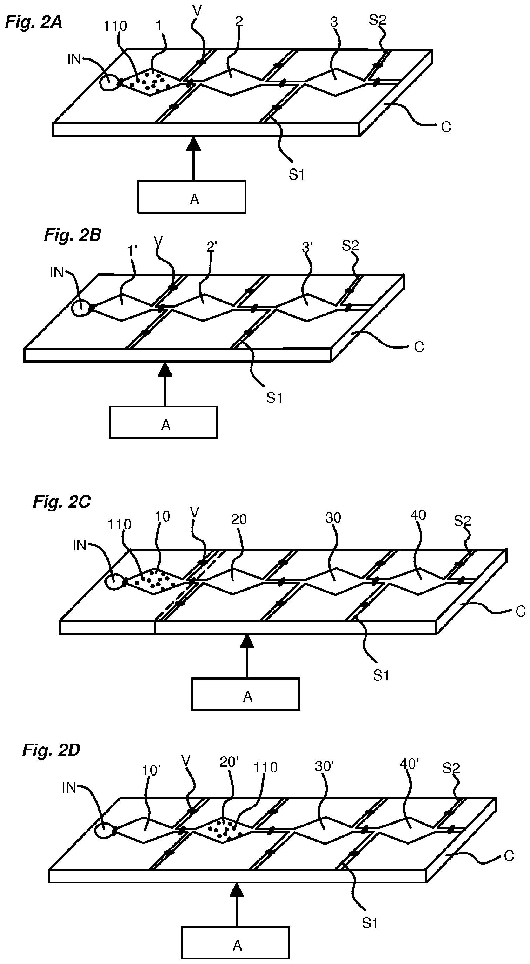

En référence aux figures 2A à 2E, selon les étapes mises en œuvre, la carte microfluidique pourra comporter trois chambres, quatre chambres ou cinq chambres.

Dans une configuration à trois chambres, représentée sur la figure 2A, la carte microfluidique C comporte :
- une première chambre 1 de déplétion/enrichissement,
- une deuxième chambre 2 de digestion, et
- une troisième chambre 3 de stabilisation.

Dans une autre configuration à trois chambres, représentée sur la figure 2B, la carte microfluidique C comporte :
- une première chambre 1' de séparation,
- une deuxième chambre 2' de digestion, et
- une troisième chambre 3' de stabilisation.

Dans une configuration à quatre chambres, représentée sur la figure 2C, la carte microfluidique C comporte :
- une première chambre 10 de séparation et/ou de déplétion/enrichissement,
- une deuxième chambre 20 de stabilisation,
- une troisième chambre 30 de digestion, et
- une quatrième chambre 40 de stabilisation.

Dans une autre configuration à quatre chambres, représentée sur la figure 2D, la carte microfluidique comporte :
- une première chambre 10' de séparation
- une deuxième chambre 20' de déplétion/enrichissement,
- une troisième chambre 30' de digestion, et
- une quatrième chambre 40' de stabilisation.

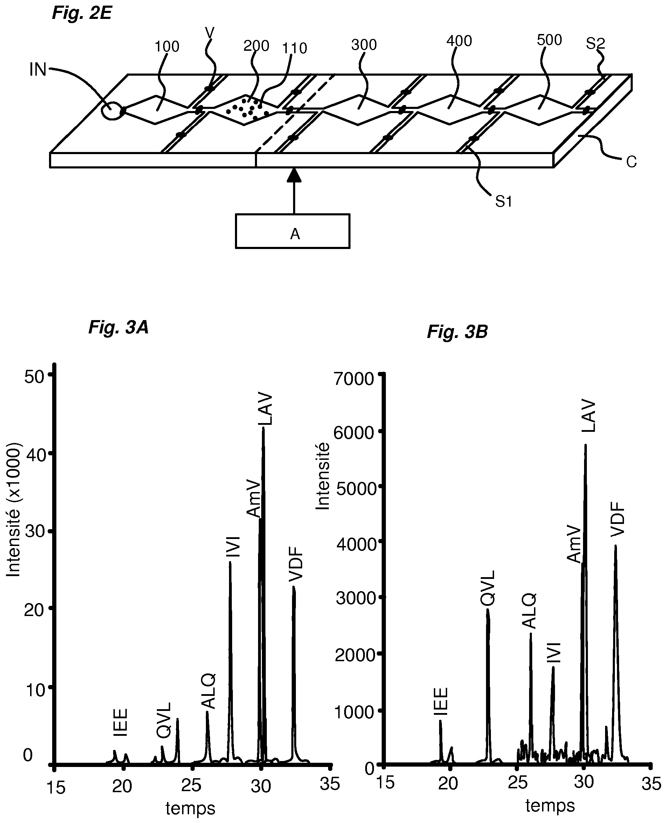

Dans une configuration à cinq chambres, représentée sur la figure 2E, la carte microfluidique comporte :
- une première chambre 100 de séparation,
- une deuxième chambre 200 de déplétion/enrichissement,
- une troisième chambre 300 de stabilisation,
- une quatrième chambre 400 de digestion, et
- une cinquième chambre 500 de stabilisation.

Pour chaque chambre, le dispositif de l'invention comporte avantageusement une vanne V située en amont et une vanne située en aval de manière à contrôler le passage de l'échantillon d'une chambre à l'autre. Chaque vanne V est avantageusement contrôlée par l'automate programmable A.

Le dispositif comporte avantageusement un canal secondaire d'injection S1 débouchant dans chaque chambre, employé notamment pour injecter des composés dans la chambre ou des solutions de lavage ou d'élution, et un canal secondaire S2 d'extraction employé par exemple pour extraire des déchets de la chambre. L'injection dans une chambre et l'extraction hors d'une chambre sont réalisées par des vannes V et des micro-pompes commandées par l'automate programmable A.

La préparation de l'échantillon pourra être réalisée dans un objectif de qualification des protéines présentes dans l'échantillon et/ou de quantification. Pour un objectif de quantification, le volume d'échantillon injecté dans le dispositif devra être calibré de manière à travailler sur un volume connu. Différents moyens de calibration pourront être envisagés. Il s'agira par exemple de commander les vannes amont et aval de la première chambre pour ajuster le volume et/ou en fixant le volume de la première chambre. La vanne aval de la première chambre est fermée et l'échantillon est alors injecté dans la chambre jusqu'à son remplissage.

En référence à la figure 1 et aux figures 2A à 2D, le dispositif microfluidique de l'invention et son utilisation pour la mise en œuvre d'un procédé de préparation d'un échantillon sanguin sont explicités ci-dessous.

La préparation de l'échantillon sanguin débute avantageusement par une première étape E1 de séparation.

Dans cette première étape E1 définie sur la figure 1, pour effectuer la séparation entre le plasma et les cellules sanguines dans l'échantillon sanguin, plusieurs solutions peuvent être envisagées, prises seule ou en combinaison entre elles :
- Une première solution consiste à réaliser la séparation dans une première chambre 1', 10,10', 100 dédiée à cette étape en utilisant un polymère, par exemple un polymère de type Dacron (marque déposée) qui permet une séparation par filtration entre le plasma et les cellules.
- Une deuxième solution consiste à employer une membrane filtrante de type Vivid (marque déposée) placée sur le chemin du canal d'injection IN ou directement sur la cartouche qui comporte initialement l'échantillon sanguin à analyser et qui est positionnée sur l'entrée du canal lors de l'injection de l'échantillon sanguin. Ce sera le cas avec une carte microfluidique réalisée selon la variante de la figure 2A ou 2C. La cartouche de prélèvement fait avantageusement partie du système de préparation de l'invention.

- Une troisième solution consiste à employer un micro-canal capillaire dans lequel l'échantillon sanguin est injecté. Ce sera le cas avec une carte microfluidique réalisée selon la variante de la figure 2A ou 2C. Ce micro-canal sera avantageusement positionné sur le canal d'injection 100.
- Une quatrième solution consiste à réaliser la séparation dans une chambre également dédiée à l'étape de déplétion ou d'enrichissement décrite ci-dessous. Ce sera le cas par exemple avec une carte microfluidique réalisée selon la variante de la figure 2C.

La deuxième étape E2 définie sur la figure 1 consiste à réaliser une déplétion des protéines majoritaires présentes dans l'échantillon plasmatique pour les capturer ou un enrichissement des protéines à analyser présentes dans cet échantillon. Il s'agira alors de simplifier l'échantillon sanguin du mieux possible et d'écarter certaines protéines majoritaires, telles que l'albumine, l'immunoglobuline de type A, M ou G, les protéines du complément, les protéines de la cicatrisation... Cette liste n'est pas limitative et une simplification de l'échantillon permettra si nécessaire de gagner en profondeur d'analyse. Bien entendu, il s'agira cependant de conserver une pluralité de protéines d'espèces distinctes.

Cette étape est préférentiellement mise en œuvre dans une chambre de déplétion ou d'enrichissement 1, 10, 20', 200 de la carte microfluidique C. L'opération de déplétion des protéines majoritaires présentes dans le plasma est par exemple réalisée en employant un support permettant la capture d'une ou de plusieurs protéines majoritaires présentes dans l'échantillon plasmatique, comme par exemple l'albumine. Comme représenté sur les figures, ce support sera par exemple des billes magnétiques 110 logées et maintenues dans leur chambre ou injectées via un canal adapté (type S1) à l'intérieur de la chambre par une commande envoyée par l'automate programmable 2. Les billes magnétiques 110 employées seront greffées avec des composants de capture des protéines majoritaires afin de les piéger dans la première chambre. Il s'agira par exemple :
- De billes magnétiques greffées avec du Cibacron Blue pour la capture de l'albumine, et/ou
- De billes magnétiques greffées avec de la protéine A pour la capture des immunoglobulines G, et/ou
- De billes greffées avec des anticorps ou ligands pour la capture spécifique des protéines majoritaires du plasma.

En variante de réalisation ou en complément aux billes 110 employées dans la chambre, le support de capture peut comporter des piliers positionnés dans la chambre, dont la surface est enduite d'un ou plusieurs des composants de capture évoqués ci-dessus. Les piliers sont fixes dans la chambre et positionnés de manière judicieuse pour que l'échantillon plasmatique s'écoule entre les piliers et viennent au contact de ceux-ci pour capter un maximum des protéines majoritaires.

En complément ou remplacement des billes 110 et/ou des piliers évoqués ci-dessus, les surfaces de la chambre peuvent être enduites de l'un des composants de capture évoqués ci-dessus afin de piéger les protéines majoritaires. La forme et la configuration de la chambre sont alors optimisées pour maximiser les surfaces d'échange entre l'échantillon plasmatique et les parois qui définissent le volume interne de la chambre. Avantageusement, pour maximiser les surfaces d'échange, la chambre est réalisée sous la forme d'un canal en serpentin.

On comprend donc que les différents moyens et agencements prévus sur la carte microfluidique C et décrits ci-dessus pour piéger les protéines majoritaires peuvent être combinés entre eux de manière judicieuse en vue d'obtenir un meilleur résultat. Il s'agira par exemple d'utiliser un canal en serpentin dont les surfaces sont enduites d'un ou plusieurs composants de capture des protéines majoritaires, ce canal débouchant dans la chambre dans laquelle sont placés des billes et/ou des piliers tels que décrits ci-dessus.

Dans le cas où une opération d'enrichissement des protéines minoritaires ciblées est réalisée en remplacement d'une opération de déplétion des protéines majoritaires présentes dans l'échantillon plasmatique, le support d'enrichissement est greffé avec des anticorps spécifiques. Comme pour la déplétion, le support peut être constitué et agencé selon l'une des variantes décrites ci-dessus pour l'opération de déplétion. Après des lavages, la fraction fixée sur le support d'enrichissement employé sera éluée vers la chambre suivante.

Une fois l'opération de déplétion ou d'enrichissement effectuée, une troisième étape E3 optionnelle consiste en une stabilisation des protéines ciblées. Cette étape de stabilisation E3 a notamment pour but de découper la préparation de l'échantillon sanguin en deux phases. Si la préparation de l'échantillon sanguin ne peut être finalisée dans un laps de temps réduit, cette étape de stabilisation peut s'avérer nécessaire. La stabilisation est avantageusement mise en œuvre dans une chambre dédiée. Cette opération de stabilisation pourra être mise en œuvre selon différentes variantes de réalisation :
- Par précipitation. Un alcool ou un acide est injecté dans la chambre puis le précipité est ensuite filtré pour ne garder que les protéines stabilisées dans la chambre.
- Par séchage, par exemple par chauffage et/ou injection d'air dans la chambre.

Cette étape de stabilisation est mise en œuvre dans une chambre de stabilisation 20, 300 présente dans une carte microfluidique telle que représentée sur les figures 2C et 2E.

Pour relancer le processus de préparation à la suite de cette étape de stabilisation, une nouvelle solubilisation des protéines présentes dans la chambre sera nécessaire. Il s'agira alors de commander l'injection d'une solution par un canal secondaire de type S1 débouchant dans la chambre de stabilisation 20, 300.

Une quatrième étape E4 de la préparation de l'échantillon sanguin consiste ensuite en une opération de digestion des protéines ciblées.

Cette étape est mise en œuvre dans une chambre 2, 2', 30, 30', 400 qui est dédiée à la digestion des protéines ciblées contenues dans l'échantillon plasmatique présent. Pour effectuer cette digestion, l'automate programmable 2 commande l'injection d'une enzyme, par exemple de type trypsine, ou un mélange d'enzymes protéolytiques via un canal secondaire de type S1 spécifique qui débouche dans ladite chambre. L'automate programmable commande ensuite un système d'agitation magnétique et un système de chauffage pour chauffer la chambre à une température déterminée afin d'assurer la réaction de digestion des protéines. En variante de réalisation ou en complément, la surface de la deuxième chambre pourra être enduite de l'enzyme ou du mélange d'enzymes nécessaire à la digestion. Dans une autre variante de réalisation ou en complément, on peut imaginer que l'enzyme ou le mélange d'enzymes soit présent dans l'espace formé par la deuxième chambre, par exemple sous une forme lyophilisée.

Une fois l'opération de digestion terminée dans la chambre dédiée, l'échantillon plasmatique qui contient alors des peptides digérés et des protéines non digérées est transféré dans une dernière chambre 3, 3', 40, 40', 500 de stabilisation. Il s'agit alors de la cinquième étape E5 du procédé de préparation de l'échantillon sanguin.

Cette chambre est dédiée à l'accrochage des peptides digérés en vue de les séparer et de les purifier. Pour cela, on utilise par exemple une colonne de chromatographie liquide, phase inverse, par exemple de type C4 ou C18 sur laquelle les peptides digérés sont retenus et peuvent être lavés puis stabilisés. Un liquide de lavage peut être injecté dans la chambre via un canal secondaire S1 débouchant dans la chambre et contrôlé par une vanne. En complément ou en variante de réalisation, il est possible de mettre en œuvre une opération d'ultra-filtration qui permet de retenir les protéines non digérées et de ne sélectionner que les peptides digérés en vue de leur stabilisation. Ce filtre sera par exemple positionné en sortie de la chambre précédente. L'établissement d'une pression pourra s'avérer nécessaire pour pousser l'échantillon à travers ce filtre. Le filtre sera ainsi adapté pour ne laisser passer que les peptides digérés et retenir les autres composés.

Lors de cette cinquième étape E5, les peptides digérés sont stabilisés dans la carte micro-fluidique. La stabilisation peut être réalisée par différentes méthodes, par exemple par fixation sur la résine type C4 ou C18, par séchage, par congélation... Pour les stabiliser, l'automate programmable A envoie une commande à un système de refroidissement ou à un système de séchage par chauffage ou par passage d'air, agencé pour agir sur la dernière chambre en vue de stabiliser les peptides présents.

Une fois les peptides stabilisés, ceux-ci peuvent être conservées dans la carte, dans la dernière chambre ou dans une chambre de stockage spécifique (non représentée).

Pour leur analyse, par exemple par spectrométrie de masse, les peptides qui ont été stabilisés (par séchage, congélation ou toute autre méthode adaptée...) sont soit élués de la carte 1 ou soit directement chargés sur une résine ou une colonne de chromatographie liquide en phase inverse de type C4 ou C18 présente dans la chambre de stabilisation.

Autrement dit, on aura ainsi les différentes configurations suivantes :
- Une carte microfluidique C telle que représentée sur la figure 2A est agencée pour mettre en œuvre les étapes E2, E4 et E5 décrites ci-dessus.
- Une carte microfluidique C de la figure 2B est agencée pour mettre en œuvre les étapes E1, E4 et E5.
- Une carte microfluidique C de la figure 2C est agencée pour mettre en œuvre les étapes E1, E3, E4 et E5.
- Une carte microfluidique C de la figure 2D est agencée pour mettre en œuvre les étapes E1, E2, E4 et E5.
- Une carte microfluidique C de la figure 2E est agencée pour mettre en œuvre les étapes E1, E2, E3, E4 et E5.

De manière avantageuse, la carte microfluidique pourra être sécable (traits pointillés P sur figures 2C et 2E), notamment lorsqu'elle comporte une chambre intermédiaire de stabilisation. Après avoir stabilisé les protéines dans cette chambre, la carte microfluidique C est sectionnée en une première partie située en amont de cette chambre de stabilisation et une deuxième partie située en aval de cette chambre. L'analyse pourra ainsi reprendre ultérieurement avec la deuxième partie de la carte comprenant les protéines stabilisées dans sa chambre de stabilisation.

De manière avantageuse, un standard protéique (avec un isotope marqué), placé avant la deuxième étape E2, ou un standard peptidique, placé après l'étape de digestion E4 et avant l'étape de stabilisation E5, peut être injecté dans la carte via un canal secondaire S1. Ce standard de qualité et quantité connues permettra notamment de mieux établir des comparaisons avec les composés présents dans l'échantillon sanguin prélevé. Ce principe est notamment décrit dans la demande de brevet publiée référencée WO2008/145763A1.

De manière avantageuse, le dispositif microfluidique de l'invention comporte des moyens pour récupérer les peptides stabilisés dans la dernière chambre de la carte C. Le dispositif comporte ainsi par exemple une membrane perforable de type septum, obturant une ouverture débouchant dans la dernière chambre de la carte. Une fois les peptides stabilisés dans la dernière chambre de stabilisation, ladite membrane est perforée pour accéder à l'intérieur de la chambre. Les peptides sont par exemple extraits de la chambre par aspiration ou tout autre moyen adapté.

Le principe de l'invention qui consiste à employer une même carte pour effectuer toutes les étapes de préparation de l'échantillon sanguin présente de nombreux avantages et permet d'obtenir des résultats particulièrement probants. On a en effet constaté que la quantité éluée et analysable des différents peptides issus de la digestion des protéines d'intérêt ciblés est bien plus importante lorsque la préparation a été effectuée de manière automatique sur un seul dispositif conforme à l'invention qu'à la suite de différentes étapes réalisées successivement manuellement. Les figures 3A et 3B permettent de se rendre compte de la qualité des résultats obtenus. L'intensité des pics de chromatographie sont bien plus importants pour les peptides analysés après une stabilisation sur le dispositif de l'invention (figure 3A) que pour des peptides analysés après une préparation manuelle classique (figure 3B).

## Revendications

1. Dispositif de préparation d'un échantillon sanguin, **caractérisé en ce qu'**il comporte une carte (C) micro-fluidique qui comporte :
- Un canal d'injection (IN) par lequel peut être injecté ledit échantillon sanguin,
- Une première chambre (1, 1', 10, 10', 100) dans laquelle débouche ledit canal d'injection, ladite première chambre étant dédiée à la mise en œuvre d'une opération de séparation et/ou d'extraction de protéines à analyser présentes dans ledit échantillon sanguin,
- Une deuxième chambre (2, 24, 30, 30', 400) reliée à ladite première chambre de manière à recevoir un premier échantillon comportant des protéines d'espèces distinctes, ladite deuxième chambre étant dédiée à la mise en œuvre d'une opération de digestion des protéines à analyser présentes en vue d'obtenir un deuxième échantillon incluant des peptides digérés et des protéines non digérées,
- Une troisième chambre (3, 3', 40, 40', 500) reliée à ladite deuxième chambre de manière à recevoir ledit deuxième échantillon incluant les peptides digérés et les protéines non digérées, ladite troisième chambre étant dédiée à la mise en œuvre d'une opération de purification et de stabilisation des peptides digérés.

2. Dispositif selon la revendication 1, **caractérisé en ce qu'**il comporte un support de déplétion présent dans la première chambre pour capturer des protéines majoritaires présentes dans ledit échantillon sanguin.

3. Dispositif selon la revendication 2, **caractérisé en ce que** le support de déplétion comporte des billes (110) présentes dans la première chambre et greffées avec un ou plusieurs composants de capture adaptés aux protéines majoritaires à capturer.

4. Dispositif selon la revendication 2 ou 3, **caractérisé en ce que** le support de déplétion comporte des piliers positionnés dans la première chambre et greffés avec un ou plusieurs composants de capture adaptés aux protéines majoritaires à capturer.

5. Dispositif selon l'une des revendications 2 à 4, **caractérisé en ce que** la première chambre est réalisée sous la forme d'un serpentin dont les surfaces fonctionnalisées forment le support de déplétion.

6. Dispositif selon la revendication 1, **caractérisé en ce qu'**il comporte un support d'enrichissement présent dans la première chambre pour fixer et enrichir les protéines à analyser présentes dans l'échantillon sanguin.

7. Dispositif selon la revendication 6, **caractérisé en ce que** le support d'enrichissement comporte des billes présentes dans la première chambre et greffées avec un ou plusieurs composants d'enrichissement adaptés aux protéines à enrichir.

8. Dispositif selon la revendication 6 ou 7, **caractérisé en ce que** le support d'enrichissement comporte des piliers positionnés dans la première chambre et greffées avec un ou plusieurs composants d'enrichissement adaptés aux protéines à enrichir.

9. Dispositif selon l'une des revendications 6 à 8, **caractérisé en ce que** la première chambre est réalisée sous la forme d'un serpentin dont les surfaces forment le support d'enrichissement.

10. Dispositif selon l'une des revendications 1 à 9, **caractérisé en ce que** la deuxième chambre comporte une surface recouverte au moins partiellement d'une enzyme ou d'un mélange d'enzymes pour la mise en œuvre de l'opération de digestion.

11. Dispositif selon l'une des revendications 1 à 9, **caractérisé en ce que** la deuxième chambre comporte un volume interne destiné à stocker une enzyme ou un mélange d'enzymes sous forme lyophilisée pour la mise en œuvre de l'opération de digestion.

12. Dispositif selon l'une des revendications 1 à 11, **caractérisé en ce qu'**il comporte une chambre intermédiaire de stabilisation située en aval de ladite première chambre et en amont de la deuxième chambre et destinée à la stabilisation des protéines extraites dans la première chambre.

13. Dispositif selon l'une des revendications 1 à 12, **caractérisé en ce qu'**il comporte une chambre initiale de séparation, située en amont de la première chambre lorsque la première chambre est dédiée à l'extraction de protéines.

14. Dispositif selon l'une des revendications 1 à 12, **caractérisé en ce qu'**il comporte des moyens de séparation agencés pour séparer le plasma et les cellules sanguines présentes dans l'échantillon sanguin, agencés en amont de la première chambre.

15. Dispositif selon l'une des revendications 1 à 14, **caractérisé en ce qu'**il comporte une colonne de chromatographie liquide en phase inverse logée dans la troisième chambre pour accrocher, stabiliser et/ou séparer les peptides digérés.

16. Dispositif selon l'une des revendications 1 à 15, **caractérisé en ce qu'**il comporte plusieurs canaux secondaires (S1, S2) réalisées dans la carte et répartis pour déboucher dans chaque chambre.

17. Système de préparation d'un échantillon sanguin comportant un automate programmable, **caractérisé en ce qu'**il comporte un dispositif de préparation d'un échantillon sanguin conforme à l'une des revendications 1 à 16 et **en ce que** l'automate programmable comporte une unité de traitement destinée à exécuter des modules logiciels adaptés pour générer des commandes à destination d'actionneurs selon une séquence déterminée en fonction des différentes étapes de préparation de l'échantillon sanguin sur ledit dispositif.

18. Procédé de préparation d'un échantillon sanguin, **caractérisé en ce qu'**il est mis en œuvre dans le dispositif de préparation d'un échantillon sanguin tel que défini dans l'une des revendications 1 à 16 et **en ce qu'**il comporte les étapes suivantes :
- Extraction dans la première chambre de protéines à analyser présentes dans ledit échantillon sanguin et/ou séparation pour séparer le plasma des cellules sanguines dans l'échantillon sanguin,
- Digestion dans la deuxième chambre des protéines à analyser présentes dans un premier échantillon comportant des protéines d'espèces distinctes à analyser en vue d'obtenir un deuxième échantillon incluant des peptides digérés et des protéines non digérées,
- Séparation dans la troisième chambre entre les peptides digérés et les protéines non digérées.

19. Procédé selon la revendication 18, **caractérisé en ce que** l'étape d'extraction est réalisée par déplétion des protéines majoritaires du plasma ou enrichissement des protéines à analyser.

20. Procédé selon la revendication 18 ou 19, **caractérisé en ce que** l'étape de stabilisation est mise en œuvre par chromatographie liquide sur une colonne de type phase inverse.

## Patentansprüche

1. Vorrichtung zur Präparation einer Blutprobe, **dadurch gekennzeichnet, dass** sie eine mikrofluidische Karte (C) umfasst, die umfasst:
- einen Injektionskanal (IN), durch den die Blutprobe injiziert werden kann,
- eine erste Kammer (1, 1', 10, 10', 100), in die der Injektionskanal mündet, wobei die erste Kammer zur Durchführung eines Vorgangs zur Separation und/oder Extraktion von zu analysierenden Proteinen, die in der Blutprobe vorhanden sind, bestimmt ist,
- eine zweite Kammer (2, 24, 30, 30', 400), die mit der ersten Kammer verbunden ist, so dass sie eine erste Probe empfängt, die Proteine unterschiedlicher Arten umfasst, wobei die zweite Kammer zur Durchführung eines Vorgangs zur Verdauung der vorhandenen zu analysierenden Proteine bestimmt ist, um eine zweite Probe zu erhalten, die verdaute Peptide und unverdaute Proteine beinhaltet,
- eine dritte Kammer (3, 3', 40, 40', 500), die mit der zweiten Kammer verbunden ist, so dass sie die zweite Probe empfängt, die die verdauten Peptide und die unverdauten Proteine beinhaltet, wobei die dritte Kammer zur Durchführung eines Vorgangs zur Reinigung und Stabilisierung der verdauten Peptide bestimmt ist.

2. Vorrichtung nach Anspruch 1, **dadurch gekennzeichnet, dass** sie ein Depletionsmedium umfasst, das in der ersten Kammer vorhanden ist, um in der Blutprobe vorhandene Mehrheitsproteine zu fangen.

3. Vorrichtung nach Anspruch 2, **dadurch gekennzeichnet, dass** das Depletionsmedium Kugeln (110) umfasst, die in der ersten Kammer vorhanden sind und auf die ein oder mehrere Fängerkomponenten gepfropft sind, die für die zu fangenden Mehrheitsproteine geeignet sind.

4. Vorrichtung nach Anspruch 2 oder 3, **dadurch gekennzeichnet, dass** das Depletionsmedium Pfeiler umfasst, die in der ersten Kammer positioniert sind und auf die ein oder mehrere Fängerkomponenten gepfropft sind, die für die zu fangenden Mehrheitsproteine geeignet sind.

5. Vorrichtung nach einem der Ansprüche 2 bis 4, **dadurch gekennzeichnet, dass** die erste Kammer in Form einer Schlange ausgeführt ist, deren funktionalisierte Oberflächen das Depletionsmedium bilden.

6. Vorrichtung nach Anspruch 1, **dadurch gekennzeichnet, dass** sie ein Anreicherungsmedium umfasst, das in der ersten Kammer vorhanden ist, um die in der Blutprobe vorhandenen zu analysierenden Proteine zu fixieren und anzureichern.

7. Vorrichtung nach Anspruch 6, **dadurch gekennzeichnet, dass** das Anreicherungsmedium Kugeln umfasst, die in der ersten Kammer vorhanden sind und auf die eine oder mehrere Anreicherungskomponenten gepfropft sind, die für die anzureichernden Proteine geeignet sind.

8. Vorrichtung nach Anspruch 6 oder 7, **dadurch gekennzeichnet, dass** das Anreicherungsmedium Pfeiler umfasst, die in der ersten Kammer positioniert sind und auf die eine oder mehrere Anreicherungskomponenten gepfropft sind, die für die anzureichernden Proteine geeignet sind.

9. Vorrichtung nach einem der Ansprüche 6 bis 8, **dadurch gekennzeichnet, dass** die erste Kammer in Form einer Schlange ausgeführt ist, deren Oberflächen das Anreicherungsmedium bilden.

10. Vorrichtung nach einem der Ansprüche 1 bis 9, **dadurch gekennzeichnet, dass** die zweite Kammer eine Oberfläche umfasst, die zumindest teilweise mit einem Enzym oder einem Enzymgemisch zur Durchführung des Verdauungsvorgangs überzogen ist.

11. Vorrichtung nach einem der Ansprüche 1 bis 9, **dadurch gekennzeichnet, dass** die zweite Kammer ein Innenvolumen umfasst, das dazu bestimmt ist, ein Enzym oder Enzymgemisch in gefriergetrockneter Form zur Durchführung des Verdauungsvorgangs zu speichern.

12. Vorrichtung nach einem der Ansprüche 1 bis 11, **dadurch gekennzeichnet, dass** sie eine Stabilisierungszwischenkammer umfasst, die stromab der ersten Kammer und stromauf der zweiten Kammer gelegen ist und zur Stabilisierung der in der ersten Kammer extrahierten Proteine bestimmt ist.

13. Vorrichtung nach einem der Ansprüche 1 bis 12, **dadurch gekennzeichnet, dass** sie eine anfängliche Separationskammer umfasst, die stromauf der ersten Kammer gelegen ist, wenn die erste Kammer zur Extraktion von Proteinen bestimmt ist.

14. Vorrichtung nach einem der Ansprüche 1 bis 12, **dadurch gekennzeichnet, dass** sie Separationsmittel umfasst, die dazu eingerichtet sind, das Plasma und die Blutzellen, die in der Blutprobe vorhanden sind, zu separieren und die stromauf der ersten Kammer eingerichtet sind.

15. Vorrichtung nach einem der Ansprüche 1 bis 14, **dadurch gekennzeichnet, dass** sie eine Umkehrphasen-Flüssigchromatographiesäule umfasst, die in der dritten Kammer untergebracht ist, um die verdauten Peptide festzuklammern, zu stabilisieren und/oder zu separieren.

16. Vorrichtung nach einem der Ansprüche 1 bis 15, **dadurch gekennzeichnet, dass** sie mehrere Nebenkanäle (S1, S2) umfasst, die in der Karte ausgeführt sind und so verteilt sind, dass sie in jede Kammer münden.

17. System zur Präparation einer Blutprobe, das eine programmierbare Steuerung umfasst, **dadurch gekennzeichnet, dass** es eine Vorrichtung zur Präparation einer Blutprobe nach einem der Ansprüche 1 bis 16 umfasst und dass die programmierbare Steuerung eine Verarbeitungseinheit umfasst, die dazu dient, geeignete Softwaremodule auszuführen, um an Aktoren gerichtete Befehle gemäß einer Sequenz zu erzeugen, die in Abhängigkeit von den verschiedenen Schritten zur Präparation der Blutprobe in der Vorrichtung bestimmt wird.

18. Verfahren zur Präparation einer Blutprobe, **dadurch gekennzeichnet, dass** es in der Vorrichtung zur Präparation einer Blutprobe wie in einem der Ansprüche 1 bis 16 definiert durchgeführt wird und dass es die folgenden Schritte umfasst:
- Extraktion, in der ersten Kammer, von in der Blutprobe vorhandenen zu analysierenden Proteinen und/oder Separation, um das Plasma von den Blutzellen in der Blutprobe zu separieren,
- Verdauung, in der zweiten Kammer, der zu analysierenden Proteine, die in einer ersten Probe vorhanden sind, die zu analysierende Proteine verschiedener Arten umfasst, um eine zweite Probe zu erhalten, die verdaute Peptide und unverdaute Proteine beinhaltet,
- Separation, in der dritten Kammer, zwischen den verdauten Peptiden und den unverdauten Proteinen.

19. Verfahren nach Anspruch 18, **dadurch gekennzeichnet, dass** der Extraktionsschritt durch Depletion der Mehrheitsproteine des Plasmas oder Anreicherung der zu analysierenden Proteine ausgeführt wird.

20. Verfahren nach Anspruch 18 oder 19, **dadurch gekennzeichnet, dass** der Stabilisierungsschritt durch Flüssigchromatographie in einer Umkehrphasensäule durchgeführt wird.

## Claims

1. Device for preparing a blood sample, **characterized in that** it includes a microfluidic card (C) which includes:
- an injection channel (IN) via which said blood sample may be injected,
- a first chamber (1, 1', 10, 10', 100) into which said injection channel emerges, said first chamber being intended for performing an operation of separation and/or extraction of proteins to be analyzed present in said blood sample,
- a second chamber (2, 24, 30, 30', 400) connected to said first chamber so as to receive a first sample comprising proteins of different species, said second chamber being intended for performing an operation of digestion of the proteins to be analyzed which are present in order to obtain a second sample including digested peptides and undigested proteins,
- a third chamber (3, 3', 40, 40', 500) connected to said second chamber so as to receive said second sample including the digested peptides and the undigested proteins, said third chamber being intended for performing an operation of purification and stabilization of the digested peptides.

2. Device according to Claim 1, **characterized in that** it includes a depletion support present in the first chamber for taking up major proteins present in said blood sample.

3. Device according to Claim 2, **characterized in that** the depletion support includes beads (110) present in the first chamber and grafted with one or more uptake components suited to the major proteins to be taken up.

4. Device according to Claim 2 or 3, **characterized in that** the depletion support includes pillars positioned in the first chamber and grafted with one or more uptake components suited to the major proteins to be taken up.

5. Device according to one of Claims 2 to 4, **characterized in that** the first chamber is made in the form of a coil whose functionalized surfaces form the depletion support.

6. Device according to Claim 1, **characterized in that** it includes an enrichment support present in the first chamber to bind and enrich the proteins to be analyzed which are present in the blood sample.

7. Device according to Claim 6, **characterized in that** the enrichment support includes beads present in the first chamber and grafted with one or more enrichment components suited to the proteins to be enriched.

8. Device according to Claim 6 or 7, **characterized in that** the enrichment support includes pillars positioned in the first chamber and grafted with one or more enrichment components suited to the proteins to be enriched.

9. Device according to one of Claims 6 to 8, **characterized in that** the first chamber is made in the form of a coil whose surfaces form the enrichment support.

10. Device according to one of Claims 1 to 9, **characterized in that** the second chamber includes a surface at least partially covered with an enzyme or a mixture of enzymes for performing the digestion operation.

11. Device according to one of Claims 1 to 9, **characterized in that** the second chamber includes an internal volume intended for storing an enzyme or a mixture of enzymes in lyophilized form for performing the digestion operation.

12. Device according to one of Claims 1 to 11, **characterized in that** it includes an intermediate stabilization chamber located downstream of said first chamber and upstream of the second chamber and intended for stabilizing the proteins extracted in the first chamber.

13. Device according to one of Claims 1 to 12, **characterized in that** it includes an initial separation chamber, located upstream of the first chamber when the first chamber is devoted to the extraction of proteins.

14. Device according to one of Claims 1 to 12, **characterized in that** it includes separation means arranged to separate the plasma and the blood cells present in the blood sample, arranged upstream of the first chamber.

15. Device according to one of Claims 1 to 14, **characterized in that** it includes a reverse-phase liquid chromatography column housed in the third chamber, to attach, stabilize and/or separate the digested peptides.

16. Device according to one of Claims 1 to 15, **characterized in that** it includes several secondary channels (S1, S2) made in the card and distributed to emerge into each chamber.

17. System for preparing a blood sample, comprising a programmable robot, **characterized in that** it includes a device for preparing a blood sample according to one of Claims 1 to 16 and **in that** the programmable robot includes a processing unit intended to run software modules suitable for generating orders intended for actuators in a given sequence as a function of the various steps for preparing the blood sample on said device.

18. Process for preparing a blood sample, **characterized in that** it is performed in the device for preparing a blood sample as defined in one of Claims 1 to 16 and **in that** it includes the following steps:
- extraction in the first chamber of proteins to be analyzed which are present in said blood sample and/or separation to separate the plasma from the blood cells in the blood sample,
- digestion in the second chamber of the proteins to be analyzed which are present in a first sample comprising proteins of different species to be analyzed for the purpose of obtaining a second sample including digested peptides and undigested proteins,
- separation in the third chamber between the digested peptides and the undigested proteins.

19. Process according to Claim 18, **characterized in that** the extraction step is performed by depletion of the major proteins of the plasma or enrichment in the proteins to be analyzed.

20. Process according to Claim 18 or 19, **characterized in that** the stabilization step is performed by liquid chromatography on a reverse-phase column.
